# EUROPÄISCHE PATENTSCHRIFT

(11) **EP 0 605 768 B1**
(45) Veröffentlichungstag und Bekanntmachung des Hinweises auf die Patenterteilung: **22.01.1997**
(21) Anmeldenummer: 93118032.7
(22) Anmeldetag: 06.11.1993
(51) Int. Cl.: C07C 29/68, C07C 29/70, C07C 31/30, C08F 10/00, D21H 25/18

(54) **Lagerstabile Lösungen von carbonisiertem Magnesiummethylat in Methanol sowie deren Herstellung und Verwendung**
Storage stable solution of carbonated magnesiummethoxide in methanol, preparation and use
Solutions stables au stockage de magnésium méthoxide carbonates en solution méthanolique, sa préparation et utilisation

(30) Priorität: 07.01.1993 DE 4300186
(43) Veröffentlichungstag der Anmeldung: 13.07.1994
(73) Patentinhaber: HÜLS AKTIENGESELLSCHAFT, 45764 Marl (DE)
(72) Erfinder: Rauleder, Hartwig, Dr., D-79618 Rheinfelden (DE); Standke, Burkhard, Dr., D-79618 Rheinfelden (DE); Kötzsch, Hans-Joachim, Dr., D-79618 Rheinfelden (DE); Schork, Reinhold, Dr., D-79618 Rheinfelden (DE)

(56) Entgegenhaltungen:
- EP-A- 0 436 801
- EP-A- 0 491 128
- US-A- 4 318 963
- US-A- 4 540 679

## Beschreibung

Die Erfindung betrifft lagerstabile Lösungen von carbonisiertem Magnesiumummethylat der Formel Mg(CH₃O)₂ (CO₂)ₙ in Methanol.

Des weiteren betrifft die Erfindung ein Verfahren zur Herstellung lagerstabiler Lösungen von carbonisiertem Magnesiummethylat der Formel Mg(CH₃O)₂ (CO₂)ₙ in Methanol durch Umsetzung von metallischem Magnesium mit Methanol und CO₂ sowie ein Verfahren zur Herstellung lagerstabiler Lösungen von carbonisiertem Magnesiummethylat der Formel Mg(CH₃O)₂ (CO₂)ₙ in Methanol durch Umsetzung von Magnesiummethylat in Methanol mit CO₂.

Außerdem betrifft die Erfindung die Verwendung der lagerstabilen Lösungen von carbonisiertem Magnesiummethylat in Methanol zur Konservierung von Papier sowie die Verwendung zur Herstellung von Katalysatoren für die Polymerisierung von Olefinen.

Magnesiumalkoholate sind in der Regel in den korrespondierenden Alkoholen wenig löslich bzw. nahezu unlöslich. Eine Ausnahme bildet Magnesiummethylat, das in Methanol eine Löslichkeit von bis zu ca. 12 Gew.-% aufweist.

Eine technisch einfache Methode, die Löslichkeit von Magnesiumalkoholaten zu erhöhen, stellt die Carbonisierung dar. In eine Suspension des Magnesiumalkoholates im korrespondierenden Alkohol wird gasförmiges, flüssiges oder festes CO₂ eingebracht. Hierbei bildet sich ein lösliches CO₂-Addukt. Das CO₂-Addukt ist im Falle von Magnesiumethylat in Ethanol mit einer Konzentration von über 30 Gew.-% löslich, hingegen ist reines Magnesiumethylat in Ethanol nahezu unlöslich.

Alkohollösliche carbonisierte Magnesiumalkoxide finden vielfache Anwendung. So können beispielsweise durch Carbonisierung in Lösung gebrachte Magnesiumalkoxide bei der Langzeitkonservierung von Papier, insbesondere von Büchern, verwendet werden und die zu diesem Zweck gemäß US-PS 3 969 549, EP-A 285 227, US-PS 4 318 963 und US-PS 3 939 091 ebenfalls eingesetzten, in der Anwendung jedoch problematischen, Zinkalkyle substituieren.

Ein weiteres Anwendungsfeld ist gemäß EP-PS 0 159 150 und EP-PS 0 236 082 die Herstellung von Katalysatoren für die Polymerisierung von Olefinen. Hierfür benötigt man z. B. sphärisches Magnesiummethylat, das beispielsweise durch Sprühtrocknung oder Fällung carbonisierter Magnesiummethylatlösungen und gegebenenfalls anschließende Decarbonisierung hergestellt werden kann.

Es ist prinzipiell bekannt, daß Lösungen carbonisierter Magnesiumalkoholate nach den beiden folgenden Reaktionsschemata hergestellt werden können, wobei einmal metallisches Magnesium mit einem Alkohol ROH und CO₂ (siehe Reaktionsgleichung 1) und zum anderen ein Magnesiumalkoxid mit CO₂ (siehe Reaktionsgleichung 2) umgesetzt werden:

Mg + 2 ROH + n CO₂ → Mg(OR)₂ (CO₂)ₙ + H₂ (1)

Mg(OR)₂ + n CO₂ → Mg(OR)₂ (CO₂)ₙ (2)

Hierbei bedeutet R eine Alkylgruppe.

So lehrt EP-PS 0 236 082 die Herstellung von carbonisierten Magnesiumalkoxiden durch Reaktion von Magnesiumalkoxiden mit CO₂ in einem Lösungsmittel. Bevorzugte Lösungsmittel sind Alkohole. Beispielsweise wird auch die Herstellung von carbonisiertem Magnesiumethylat durch Umsetzung von Magnesiumethylat mit CO₂ in Ethanol als Lösungsmittel offenbart.

Fieser und Fieser beschreiben in "Reagents for Organic Synthesis", Vol. 1, Seite 631, die Herstellung von carbonisiertem Magnesiummethylat auf zwei verschiedenen Wegen:
Zum einen wird eine Suspension von Magnesiummethylat in Methanol mit CO₂ umgesetzt.
Zum anderen läßt man Magnesiumspäne mit Methanol zu Magnesiummethylat reagieren. Nachdem der Methanol teilweise bei 50 °C und Unterdruck abgezogen worden ist, wird Dimethylformamid als Lösungsmittel zugesetzt und CO₂ in diese Lösung eingeleitet. Der restliche Methanol wird abdestilliert, und man erhält eine schwach gelbe Lösung von carbonisiertem Magnesiummethylat in Dimethylformamid.

In EP-PS 0 159 150 wird über die Verwendung von Lösungen carbonisierter Magnesiumalkoholate in Alkoholen zur Herstellung aktiver Katalysatorträgermaterialien für die Olefinpolymerisation berichtet. Die verwendeten Lösungen enthalten 10 bis 80 Gew.-% Magnesiumalkoholat, 0,1 bis 4 mol CO₂ pro mol Magnesium und werden durch Umsetzung von Magnesiumalkoholat, dispergiert in Alkohol, mit gasförmigem CO₂ hergestellt.

Die alkoholischen Lösungen carbonisierter Magnesiumalkoxide gemäß dem Stand der Technik sind jedoch nur kurzzeitig verwendbar, da bei Lagerung selbst in dicht verschlossenen Gefäßen schon nach einigen Tagen Verfärbungen, Ausfällungen oder Gelierungen auftreten können.

Die technische Verwendung solcher Lösungen ist erheblich eingeschränkt: Einerseits können verfärbte Lösungen beispielsweise nicht für die Langzeitkonservierung von Büchern verwendet werden, andererseits sind Lösungen, bei denen die Gefahr unkontrollierter Ausfällungen besteht, für die Herstellung von Katalysatoren auf Basis Magnesiumalkoxid unbrauchbar.

Der Erfindung liegt die Aufgabe zugrunde, lagerstabile Lösungen von carbonisiertem Magnesiummethylat herzustellen, die auch über einen längeren Zeitraum nahezu farblos bleiben und weder Ausfällungen noch Gelierung zeigen.

Es wurde nun überraschenderweise gefunden, daß durch Einstellung definierter Magnesium- und CO₂-Konzentrationen in den erfindungsgemäßen Lösungen von carbonisiertem Magnesiummethylat in Methanol Verfärbungen, Ausfällungen und Gelierungen auch während der Lagerung über lange Zeiträume nicht auftreten.

Gegenstand der vorliegenden Erfindung sind daher lagerstabile Lösungen von carbonisiertem Magnesiummethylat der Formel Mg(CH₃O)₂ (CO₂)ₙ in Methanol, die dadurch gekennzeichnet sind, daß der Magnesiumgehalt der Lösung 0,1 bis 8 Gew.-%, bezogen auf die gesamte Lösung, und der CO₂-Gehalt n gleich 1 bis 2,2 beträgt.

Des weiteren ist Gegenstand der vorliegenden Erfindung ein Verfahren zur Herstellung lagerstabiler Lösungen von carbonisiertem Magnesiummethylat der Formel Mg(CH₃O)₂ (CO₂)ₙ in Methanol durch Umsetzung von metallischem Magnesium mit Methanol und CO₂, das dadurch gekennzeichnet ist, daß der Magnesiumgehalt der Lösung auf Werte von 0,1 bis 8 Gew.-%, bezogen auf die gesamte Lösung, und der CO₂-Gehalt auf Werte von n gleich 1 bis 2,2 eingestellt werden sowie
ein Verfahren zur Herstellung lagerstabiler Lösungen von carbonisiertem Magnesiummethylat der Formel Mg(CH₃O)₂ (CO₂)ₙ in Methanol durch Umsetzung von Magnesiummethylat in Methanol mit CO₂, das dadurch gekennzeichnet ist, daß der Magnesiumgehalt der Lösung auf Werte von 0,1 bis 8 Gew.-%, bezogen auf die gesamte Lösung, und der CO₂-Gehalt auf Werte von n gleich 1 bis 2,2 eingestellt werden.

Außerdem ist Gegenstand der vorliegenden Erfindung die Verwendung der lagerstabilen Lösungen von carbonisiertem Magnesiummethylat in Methanol nach Anspruch 1 zur Konservierung von Papier sowie die Verwendung der lagerstabilen Lösungen von carbonisiertem Magnesiummethylat in Methanol nach Anspruch 1 zur Herstellung von Katalysatoren für die Polymerisierung von Olefinen.

Bei der Herstellung der erfindungsgemäßen Lösungen von carbonisiertem Magnesiummethylat dient als Magnesiumquelle entweder metallisches Magnesium (siehe Gleichung 1) oder Magnesiummethylat (siehe Gleichung 2). Die Verwendung von metallischem Magnesium ist wirtschaftlich vorteilhafter, da Magnesiummethylat üblicherweise aus Magnesiummetall durch Umsetzung mit Methanol gewonnen wird und somit ein Reaktionsschritt entfällt. Weiterhin lassen sich bei der Umsetzung von metallischem Magnesium mit Methanol und CO₂ gemäß Gleichung (1) bezüglich der Farbqualität hochwertigere Produkte erzeugen. Man erhält also bei der Umsetzung von metallischem Magnesium mit Methanol und CO₂ erfindungsgemäße Lösungen, die im Vergleich mit den aus Magnesiummethylat und CO₂ gemäß Gleichung (2) hergestellten erfindungsgemäßen Lösungen etwas geringere Farbzahlen, gemessen nach der Meßmethode von Gardner, aufweisen.

Aufgrund der Tatsache, daß bei der Umsetzung von metallischem Magnesium mit Methanol und CO₂ die Oberfläche des Magnesiums wegen der Löslichkeit des carbonisierten Magnesiummethylats ständig freiliegt, erfolgt keine Passivierung der Metalloberfläche durch die Bildung einer unlöslichen Grenzschicht zwischen Metall und Methanol. Man ist daher bei der Herstellung der erfindungsgemäßen Lösungen gemäß Gleichung (1) nicht auf die Verwendung von oberflächenreichem Magnesiummaterial, wie z. B. Magnesiumspänen oder Magnesiumgrieß, angewiesen, wie es beispielsweise bei der technischen Herstellung von Magnesiummethylat benötigt wird, sondern kann preislich günstigere und sicherheitstechnisch unproblematerischer zu handhabende Magnesiumblockware einsetzen. Weiterhin benötigt man zum Starten der Reaktion keinerlei Hilfssubstanzen, wie beispielsweise Quecksilbersalze (siehe Liebigs Annalen der Chemie 444, 236, 1925), so daß die erreichbare Produktreinheit bei Einsatz von Magnesiummetall gemäß Gleichung (1) im Vergleich mit der bei Einsatz von Magnesiummethylat gemäß Gleichung (2) etwas größer ist.

Die Carbonisierung kann durch Einleitung von gasförmigem CO₂ oder durch Zugabe von flüssigem oder festem CO₂ in das Reaktionsgemisch aus Methanol und Magnesium gemäß Gleichung (1) bzw. aus Methanol und Magnesiummethylat gemäß Gleichung (2) erfolgen.

Es kann also der CO₂-Gehalt n der erfindungsgemäßen Lösung durch Zudosieren von gasförmigem, flüssigem oder festem CO₂ in das Gemisch eingestellt werden.

Eine andere Variante ist, daß der CO₂-Gehalt n der erfindungsgemäßen Lösung durch thermisches Austreiben von CO₂ aus einer einen Überschuß an CO₂ enthaltenden Lösung von carbonisiertem Magnesiummethylat in Methanol eingestellt werden kann.

Die Menge des eingebrachten CO₂ ist einfach über Massenbilanzierung durch Gewichtszunahme kontrollierbar. Durch Abdestillieren von Methanol aus einer Lösung von carbonisiertem Magnesiummethylat in Methanol oder durch Eindosieren von Methanol in eine Lösung von carbonisiertem Magnesiummethylat in Methanol ist die Magnesiumkonzentration der erfindungsgemäßen Lösung auf einfache Weise einstellbar. Bevorzugt wird der Magnesiumgehalt der erfindungsgemäßen Lösung durch destillative Entfernung von Methanol aus einer einen Überschuß an Methanol enthaltenden Lösung von carbonisiertem Magnesiummethylat in Methanol eingestellt.
Aufgrund der Hydrolyse- und Oxidationsempfindlichkeit der erfindungsgemäßen Lösungen sind sämtliche Operationen unter Ausschluß von Luft und Feuchtigkeit durchzuführen.

Lösungen von carbonisiertem Magnesiummethylat in Methanol mit zu hoher Magnesiumkonzentration, d. h. mit einer Magnesiumkonzentration von größer als ca. 8,9 Gew.-%, neigen zu Ausfällungen bzw. Gelierung. Überraschenderweise ist die Neigung zur Auskristallisation von Niederschlägen bzw. Gelierung nicht wie bei Lösungen von carbonisiertem Magnesiumethylat in Ethanol so stark abhängig von der zur Carbonisierung eingesetzten Menge an CO₂. So kann beispielsweise eine gegen Ausfällungen stabile, erfindungsgemäße Lösung von Magnesiummethylat in Methanol mit einem Magnesiumgehalt von mehr als 5 Gew.-% und einem CO₂-Gehalt n von mehr als 2 hergestellt werden. Bei einer Lösung von carbonisiertem Magnesiumethylat in Ethanol kristallisiert bei gleicher Magnesium- und CO₂-Konzentration in wenigen Wochen ein Niederschlag aus. Ebenfalls überraschend ist die Tatsache, daß bei erfindungsgemäßen Lösungen von carbonisiertem Magnesiummethylat in Methanol ein geringer CO₂-Gehalt von n gleich 1 bis 1,2 bei Lagerung innerhalb von Wochen nicht zu Verfärbungen, wie beispielsweise bei Lösungen von carbonisiertem Magnesiumethylat in Ethanol, führt.

Stellt man die Magnesiumkonzentration auf Werte von 0,1 bis 8 Gew.-% und den CO₂-Gehalt n auf Werte von 1 bis 2,2 ein, so erhält man die gegenüber Ausfällungen und Farbveränderungen stabilen erfindungsgemäßen Lösungen.

Ethanolische Lösungen von carbonisiertem Magnesiumethylat mit einem Magnesiumgehalt von 3,6 Gew.-%, hergestellt durch Carbonisierung von handelsüblichem Magnesiumethylat (Normalkorn, Hersteller: Hüls AG) in Ethanol weisen kurz nach Herstellung eine Farbzahl von 2-3 (gemessen nach "Gardner") auf. Für erfindungsgemäße Lösungen, hergestellt durch Carbonisierung von Magnesiummethylat in Methanol, erhält man hingegen bei gleichen Konzentrationsverhältnissen überraschenderweise Farbzahlen von nicht mehr als 2 (gemessen nach "Gardner"). Verwendet man zur Herstellung anstelle von Magnesiummethylat als Ausgangsmaterial Magnesiumblockware, so erhält man bei Lösungen gleicher Magnesium- und CO₂-Konzentrationen Farbzahlen von < 1 (gemessen nach "Gardner"). Setzt man also als Magnesiumquelle metallisches Magnesium bei der Herstellung der erfindungsgemäßen Lösungen ein, so erhält man im Hinblick auf Reinheit und Farbqualität etwas bessere Produkte als bei Einsatz von Magnesiummethylat als Ausgangsmaterial.

Die Erfindung wird durch die folgenden Beispiele näher erläutert:

### Beispiel 1

Herstellung einer lagerstabilen Lösung von carbonisiertem Magnesiummethylat mit hohem CO₂-Gehalt aus Magnesiummethylat und CO₂ in Methanol.

In einem 1 l Dreihalskolben, ausgerüstet mit Rückflußkühler, Gaseinleitungsrohr, Stickstoffüberlagerung und KPG-Flügelrührer werden 470 g Methanol vorgelegt und anschließend 135 g Magnesiummethylat (Hersteller: Hüls AG) unter Stickstoffbeschleierung zugesetzt. Unter Rühren wird CO₂ eingeleitet. Die CO₂-Aufnahme wird über Gewichtskontrolle kontinuierlich verfolgt. Die Temperatur im Reaktionsraum steigt kontinuierlich an. Bei Aufnahme von 139 g CO₂, entsprechend einem CO₂-Gehalt n gleich 2,02, wird die Reaktion beendet. Die Temperatur erreicht einen Maximalwert von 64 °C. Die so erzeugte Lösung ist leicht gelb gefärbt und besitzt eine Farbzahl von 1 bis 2 (gemessen nach "Gardner") und einen Magnesiumgehalt von 5,1 Gew.-%. Nach 6 Wochen Lagerung in dichtverschlossener Glasflasche tritt weder eine Verfärbung noch Ausfällung oder Gelierung auf.

### Beispiel 2

Herstellung einer lagerstabilen Lösung von carbonisiertem Magnesiummethylat mit niedrigem CO₂-Gehalt aus Magnesiummethylat und CO₂ in Methanol.

In einem 1 l Dreihalskolben, ausgerüstet mit Rückflußkühler, Gaseinleitungsrohr, Stickstoffüberlagerung und KPG-Flügelrührer werden 470 g Methanol vorgelegt und anschließend 135 g Magnesiummethylat (Hersteller: Hüls AG) unter Stickstoffbeschleierung zugesetzt. Unter Rühren wird CO₂ eingeleitet. Die CO₂-Aufnahme wird über Gewichtskontrolle kontinuierlich verfolgt. Die Temperatur im Reaktionsraum steigt kontinuierlich an. Bei Aufnahme von 78 g CO₂, entsprechend einem CO₂-Gehalt n gleich 1,13, wird die Reaktion beendet. Die Temperatur erreicht einen Maximalwert von 64 °C. Die so erzeugte Lösung ist leicht gelb gefärbt und besitzt eine Farbzahl von 1 bis 2 (gemessen nach "Gardner") und einen Magnesiumgehalt von 5,5 Gew.-%. Nach 6 Wochen Lagerung in dichtverschlossener Glasflasche tritt weder eine Verfärbung noch Ausfällung oder Gelierung auf.

### Beispiel 3 (Vergleichsbeispiel)

Zur Einstellung einer höheren Magnesiumkonzentration werden 500 g der Lösung aus Beispiel 1 am Rotationsverdampfer bei einer von 20 auf 30 °C steigenden Sumpftemperatur und bei einem von 220 auf 75 mbar absolut sinkenden Druck in einem Zeitraum von ca. 1,5 Stunden eingeengt. Die Lösung verliert hierbei ca. 38 g CO₂ und 150 g Methanol. Der CO₂-Gehalt n der viskosen Lösung beträgt 1,19 und der Magnesiumgehalt 8,9 Gew.-%. Nach ca. 4 Tagen Lagerung erfolgt zunächst die Auskristallisation eines Niederschlages. Nach ca. 3 Wochen geliert die Lösung vollständig.

### Beispiel 4

Herstellung einer lagerstabilen Lösung von carboniertem Magnesiummethylat mit hohem Magnesiumgehalt aus Magnesiummethylat und CO₂ in Methanol.

Zur Einstellung einer höheren Magnesiumkonzentration werden 500 g der Lösung aus Beispiel 1 am Rotationsverdampfer bei einer von 20 auf 30 °C steigenden Sumpftemperatur und bei einem von 220 auf 120 mbar absolut sinkenden Druck in einem Zeitraum von ca. 1 Stunde eingeengt. Die Lösung verliert hierbei ca. 35 g CO₂ und 125 g Methanol. Der CO₂-Gehalt n der viskosen Lösung beträgt 1,26 und der Magnesiumgehalt 7,5 Gew.-%.

Nach 6 Wochen Lagerung in dicht verschlossener Glasflasche tritt weder eine Verfärbung noch Ausfällung oder Gelierung auf.

### Beispiel 5

Herstellung einer lagerstabilen Lösung von carbonisiertem Magnesiummethylat aus metallischem Magnesium und CO₂ in Methanol.

In einem 4 l Doppelmantel-Dreihalskolben, ausgerüstet mit Rückflußkühler, Gaseinleitungsrohr, Stickstoffüberlagerung und KPG-Flügelrührer, werden 2 000 g Methanol vorgelegt und anschließend 142 g Magnesiumblockware (Hersteller: Normag), die auf eine Kantenlänge von 9 cm x 2 cm x 2 cm zurechtgesägt worden ist, unter Stickstoffbeschleierung in die Reaktionsapparatur mittels Edelstahldraht so eingehängt, daß sie vollständig in Methanol eintaucht. Unter Rühren wird CO₂ eingeleitet. Die CO₂-Aufnahme wird über Gewichtskontrolle kontinuierlich verfolgt. Mittels Thermostatbeheizung wird leichter Rückfluß eingestellt. Nach Aufnahme von 436 g CO₂, entsprechend einem CO₂-Gehalt n gleich 1,75, hat sich das Magnesium unter Wasserstoffentwicklung gelöst. Der Magnesiumgehalt der Lösung beträgt 5,5 Gew.-%.
Die resultierende Lösung ist klar und weist eine Farbzahl von < 1 (gemessen nach "Gardner") auf. Nach 6 Wochen Lagerung in dichtverschlossener Glasflasche tritt weder eine Verfärbung noch Ausfällung oder Gelierung auf.

## Patentansprüche

1. Lagerstabile Lösungen von carbonisiertem Magnesiummethylat der Formel Mg(CH₃O)₂ (CO₂)ₙ in Methanol,
dadurch gekennzeichnet,
daß der Magnesiumgehalt der Lösung 0,1 bis 8 Gew.-%, bezogen auf die gesamte Lösung, und der CO₂-Gehalt n gleich 1 bis 2,2 beträgt.

2. Verfahren zur Herstellung lagerstabiler Lösungen von carbonisiertem Magnesiummethylat der Formel Mg(CH₃O)₂ (CO₂)ₙ in Methanol nach Anspruch 1 durch Umsetzung von metallischem Magnesium mit Methanol und CO₂,
dadurch gekennzeichnet,
daß der Magnesiumgehalt der Lösung auf Werte von 0,1 bis 8 Gew.-%, bezogen auf die gesamte Lösung, und der CO₂-Gehalt auf Werte von n gleich 1 bis 2,2 eingestellt werden.

3. Verfahren zur Herstellung lagerstabiler Lösungen von carbonisiertem Magnesiummethylat der Formel Mg(CH₃O)₂ (CO₂)ₙ in Methanol nach Anspruch 1 durch Umsetzung von Magnesiummethylat in Methanol mit CO₂,
dadurch gekennzeichnet,
daß der Magnesiumgehalt der Lösung auf Werte von 0,1 bis 8 Gew.-%, bezogen auf die gesamte Lösung, und der CO₂-Gehalt auf Werte von n gleich 1 bis 2,2 eingestellt werden.

4. Verfahren nach den Ansprüchen 2 oder 3,
dadurch gekennzeichnet,
daß der CO₂-Gehalt n der Lösung durch Zudosieren von gasförmigem, flüssigem oder festem CO₂ in das Gemisch eingestellt wird.

5. Verfahren nach den Ansprüchen 2 oder 3,
dadurch gekennzeichnet,
daß der CO₂-Gehalt n der Lösung durch thermisches Austreiben von CO₂ aus einer einen Überschuß an CO₂ enthaltenden Lösung von carbonisiertem Magnesiummethylat in Methanol eingestellt wird.

6. Verfahren nach den Ansprüchen 2 bis 5,
dadurch gekennzeichnet,
daß der Magnesiumgehalt der Lösung durch destillative Entfernung von Methanol aus einer einen Überschuß an Methanol enthaltenden Lösung von carbonisiertem Magnesiummethylat in Methanol eingestellt wird.

7. Verwendung der lagerstabilen Lösungen von carbonisiertem Magnesiummethylat in Methanol nach Anspruch 1 zur Konservierung von Papier.

8. Verwendung der lagerstabilen Lösungen von carbonisiertem Magnesiummethylat in Methanol nach Anspruch 1 zur Herstellung von Katalysatoren für die Polymerisierung von Olefinen.

## Claims

1. Storage-stable solutions of carbonated magnesium methylate of the formula Mg(CH₃O)₂ (CO₂)ₙ in methanol, characterized in that the magnesium content of the solution is 0.1 to 8% by weight, relative to the total solution, and the CO₂ content is n equal to 1 to 2.2.

2. Process for preparing storage-stable solutions of carbonated magnesium methylate of the formula Mg(CH₃O)₂ (CO₂)ₙ in methanol according to Claim 1 by reacting metallic magnesium with methanol and CO₂, characterized in that the magnesium content of the solution is adjusted to values of 0.1 to 8% by weight, relative to the total solution, and the CO₂ content to values or n equal to 1 to 2.2.

3. Process for preparing storage-stable solutions of carbonated magnesium methylate of the formula Mg(CH₃O)₂ (CO₂)ₙ in methanol according to Claim 1 by reacting magnesium methylate in methanol with CO₂, characterized in that the magnesium content of the solution is adjusted to values of 0.1 to 8% by weight, relative to the total solution, and the CO₂ content to values of n equal to 1 to 2.2.

4. Process according to Claims 2 or 3, characterized in that the CO₂ content n of the solution is adjusted by metered addition of gaseous, liquid or solid CO₂ to the mixture.

5. Process according to claims 2 or 3, characterized in that the CO₂ content n of the solution is adjusted by thermally expelling CO₂ from a solution of carbonated magnesium methylate in methanol containing an excess of CO₂.

6. Process according to Claims 2 to 5, characterized in that the magnesium content of the solution is adjusted by distillative removal of methanol from a solution of carbonated magnesium methylate in methanol containing an excess of methanol.

7. Use of the storage-stable solutions of carbonated magnesium methylate in methanol according to Claim 1 for preserving paper.

8. Use of the storage-stable solutions of carbonated magnesium methylate in methanol according to Claim 1 for preparing catalysts for the polymerization of olefins.

## Revendications

1. Solutions stables au stockage de méthylate de magnésium carbonisé de formule Mg(CH₃O)₂ (CO₂)ₙ dans le méthanol,
caractérisées en ce que
la teneur en magnésium de la solution s'élève de 0,1 à 8 % en poids, rapporté à la solution totale, et la teneur en CO₂ exprimée par n est égale à 1 à 2,2.

2. Procédé de fabrication de solutions stables au stockage de méthylate de magnésium carbonisé de formule Mg(CH₃O)₂ (CO₂)ₙ dans le méthanol selon la revendication 1, par réaction de magnésium métallique avec le méthanol et CO₂,
caractérisé en ce que
la teneur en magnésium de la solution est ajustée à des valeurs allant de 0,1 à 8 % en poids, rapporté à la solution totale, et la teneur en CO₂ est ajustée à des valeurs de n égales à 1 à 2,2.

3. Procédé de fabrication de solutions stables au stockage de méthylate de magnésium carbonisé de formule Mg(CH₃O)₂ (CO₂)ₙ dans le méthanol selon la revendication 1, par réaction de magnésium métallique avec le méthanol et CO₂,
caractérisé en ce que
la teneur en magnésium de la solution est ajustée à des valeurs allant de 0,1 à 8 % en poids rapporté à la solution totale et en ce que la teneur en CO₂ est ajustée à des valeurs de n égales à 1 à 2,2.

4. Procédé selon les revendications 2 ou 3,
caractérisé en ce que
la teneur en CO₂ n de la solution est ajustée par l'addition dosée de CO₂ gazeux, liquide ou solide dans le mélange.

5. Procédé selon les revendications 2 ou 3,
caractérisé en ce que
la teneur en CO₂ n de la solution est ajustée par évacuation par la chaleur de CO₂ à partir d'une solution contenant un excès de CO₂, de méthylate de magnésium carbonisé dans le méthanol.

6. Procédé selon les revendications 2 à 5,
caractérisé en ce que
la teneur en magnésium de la solution est ajustée par élimination par distillation du méthanol d'une solution contenant un excès de méthanol, de méthylate de magnésium carbonisé dans le méthanol.

7. Utilisation des solutions stables au stockage de méthylate de magnésium carbonisé dans le méthanol selon la revendication 1 en vue de la conservation du papier.

8. Utilisation de solutions stables au stockage de méthylate de magnésium carbonisé dans le méthanol, selon la revendication 1, en vue de la production de catalyseurs pour la polymérisation des oléfines.
